# EUROPEAN PATENT SPECIFICATION

(11) **EP 1 480 568 B1**
(45) Date of publication and mention of the grant of the patent: **10.09.2008**
(21) Application number: 03704822.0
(22) Date of filing: 28.02.2003
(51) Int. Cl.: A61B 17/66

(54) **Osteogenesis device**
Vorrichtung zur Osteosynthese
Dispositif d'ostéosynthèse

(30) Priority: 01.03.2002 GB 0204783
(43) Date of publication of application: 01.12.2004
(73) Proprietor: Computergen Implants Limited, Doncaster DN5 7BW (GB)
(72) Inventor: PECKITT, Ninian, Doncaster DN5 7BW (GB)
(74) Representative: Gilholm Harrison Ltd
(86) International application number: PCT/GB2003/000814
(87) International publication number: WO 2003/073947

(56) References cited:
- EP-A- 1 118 309
- US-A- 5 700 263
- PECKITT N.: "Orofacial Regeneration Customised Distraction Osteogenesis The Spiral Vector Distractor" INTERNET ARTICLE, [Online] XP002244422 Retrieved from the Internet: <URL:http://www.maxfac.com/CustomDistract. htm> [retrieved on 2003-06-16]
- DATABASE WPI Section PQ, Week 199316 Derwent Publications Ltd., London, GB; Class P31, AN 1993-132245 XP002244423 -& SU 1 731 199 A (AZARKH V N), 7 May 1992 (1992-05-07)
- DATABASE WPI Section PQ, Week 199743 Derwent Publications Ltd., London, GB; Class P31, AN 1997-465253 XP002244424 -& JP 09 215699 A (NAGOYA NISHIKO SEISAKUSHO KK), 19 August 1997 (1997-08-19)

## Description

This invention relates to an osteogenesis method and device.

Distraction osteogenesis ("DO") was originally described by Ilizarov in the 1950's in the lengthening of limb bones by the manipulation of callus following osteotomy, with external pins passing through the skin. DO was later adapted for advancement of the facial bones using both internal screw devices and external pin distraction devices. These devices were developed as a simpler alternative to complex composite flap reconstruction of the facial skeleton, which involve long and complex surgical multi-sited and multi-staged procedures. In particular, the area of the chin is technically very difficult to reconstruct.

All distraction devices to date have a problem, with precise choice of vector leading to deformity, and malocclusion. External pins cause skin necrosis and scarring as they are pulled through the tissue with their attached bone. These devices also have a low mechanical advantage leading to bending and fracture of transcutaneous pins. Furthermore, reconstruction of the curved vector of the chin is difficult especially with external pins and the associated frames and this leads to unwarranted soft tissue damage.

US Patent No. 5,700,263 describes a distraction device for osteosynthesis which includes a first member which is telescopically housed within a second member. The first and second members are attached to first and second bone segments, respectively. The device includes a ratchet wheel having teeth which engage with teeth of the first member. Rotation of the ratchet wheel causes the first member to telescopically extend from its second member housing in an arcuate path.

Russian patent no. 1731199 describes a compression-distraction apparatus for jaw osteoplasty which consists of a flexible plate attached to the bone of a jaw by screws. A mobile plate is fixed onto the flexible palate and the mobile plate is moveable by winding a flexible string attached to the plate onto a drum.

According to the present invention, there is provided a spiral distraction device comprising anchoring means for attachment of the device to each side of a patient's existing mandible, bar means conjoined to and intermediate between each side of the patients' mandible and adapted to attach to and transport moveable bone pieces on each side of the patient's mandible towards each other in a spiral vector that is larger than the patient's anatomical mandible contour, and means to action the intermediate bar means to effect said transport.

Preferably the spiral vector is a spiral curve vector; such a curved vector preferably produces a class 111 or mandible prognathism contour.

By spiralling the moving bone in opposite rotations, the moveable bone pieces can be transported to almost overlap with each other at or near the mid-line position. Removal of the distraction device from the patient once bone-transportation is complete allows the surgeon to 'bend' the bone pieces back towards each other to achieve bony overlap and thereafter be conjoined in a wholly or substantially regular line across the midline.

Using rapid prototyping and engineering assisted surgery techniques, preoperative planning can ensure a normal degree of mandibular prognathism. This is achieved with the conversion of a prognathic Class III mandibular profile at the endpoint of distraction to a normal Class I mandibular profile following removal of the distraction device and bending of the intervening callous to achieve bony overlap in the midline.

In one embodiment of the present invention, the intermediate bar means includes a capstan means through or around which the relevant part of the bar means and/or means for attachment is wound or otherwise rotated to effect the transportation action. Also preferably, the capstan includes one or more key attachment means such as a suitable aperture in which a bar, key etc, can be permanently or removably inserted.

The intermediate bar means preferably includes one or more spiral vector bars whose distal ends are attachable to the mandible attachment means. The attachment means act as anchors.

The distraction device includes one or more shuttles which may be adapted to wholly or substantially cut or cleave through patient's tissue to assist in transportation of the moving bone pieces therethrough.

The spiral action of each vector could equally result in the right hand bone pieces being transported to a position above the left hand bone piece at or near the midline position, or vice versa.

The spiral curve vector of each bone piece is preferably wholly or substantially a parabolic vector shape.

The method of attachment of the device to a mandible, and method of attachment of bone pieces together once transported, are well known in the art. Such means includes screws, etc.

There is provided a method of forming a mandible for a patient using a spiral distraction device as hereinbefore defined, comprising the steps of;
attaching anchoring means to each side of a patient's existing mandible;
conjoining the intermediate bar means to the anchoring means and to moveable bone pieces on each side of the patient;
activating the intermediate bar means to transport the bone pieces towards each other in a spiral vector that is larger that the patient anatomical mandible contour;
removing the distraction device at the end point of distraction; and
conjoining the bone pieces.

Preferably, the bone pieces are transported towards each other by bending the bone pieces backwards so as to achieve bony overlap. This is facilitated by virtue of the malleability of adjacent callous in the area of distraction.

The distraction device is removed at the end point of distraction, i.e. when the shuttles reach their end point of movement by contact with the capstan.

Preferably, the bone pieces are transported through a number of discrete steps.

The spiral distraction device of the present invention provides; accurate presurgical planning with selection of a predetermined vector using rapid prototyping reverse engineering and Engineering Assisted Surgery^{®} Principles which facilitate the translation of the surgical plan into the patient;
improved Mechanical Advantage, with less force required for distraction and less soft tissue injury;
an internal device using cord or chain distraction techniques and an external capstan/key mechanism; easy assembly and disassembly;
a mechanism to facilitate bony union between the distracted segments with bony overlap using a lag/position screw system which may also double as an overdenture attachment system; and
spiral distraction vectors which permit easy removal of the device from the patient with a ring hammer device.

The use of the present device is also associated with the reduced surgical trauma and operating time and has applications in other areas of the skeleton.

The present invention is useable to reconstruct any bit of the lower jaw, i.e. the body of the mandible, as well as the chin. In that regard, the spiral vector may be a 'straight' spiral vector, i.e. the bone spirals along a substantially straight line, as well as a curved spiral vector for the front of the chin.

An embodiment of the present invention will now be described by way of example only and with reference to the accompanying photographic drawings in which:

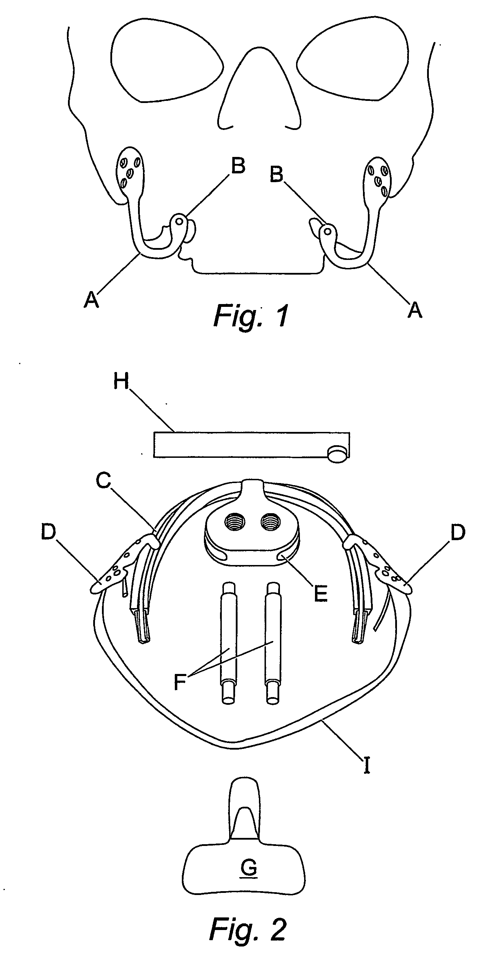

Figure 1 shows anchoring devices planted on a stereoscopic lithographic model;
Figure 2 shows parts of a distraction device of the present invention;

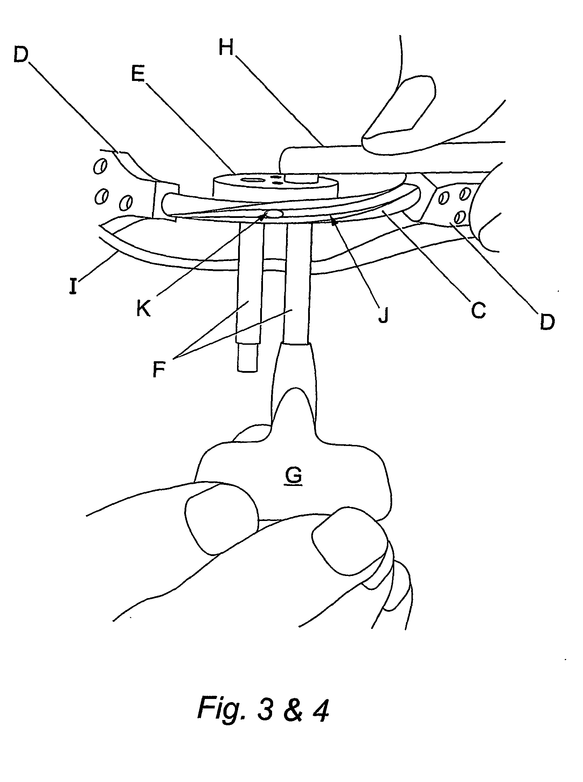

Figures 3 and 4 show assembly of the device;

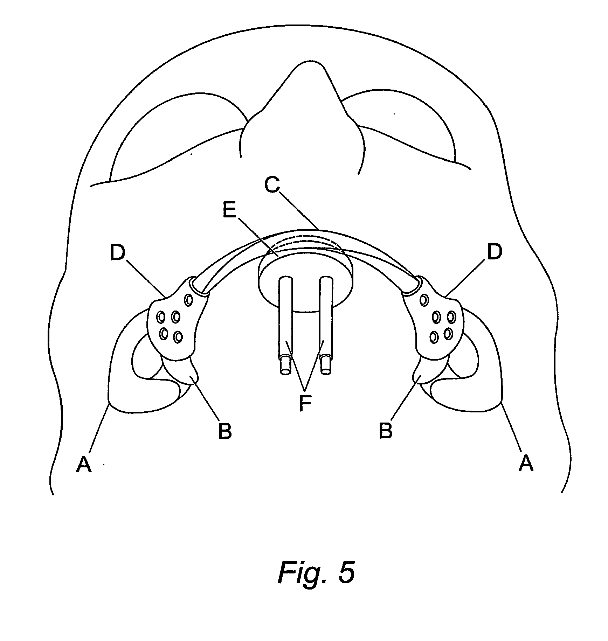

Figure 5 shows the assembled device on a lithographic model from below.

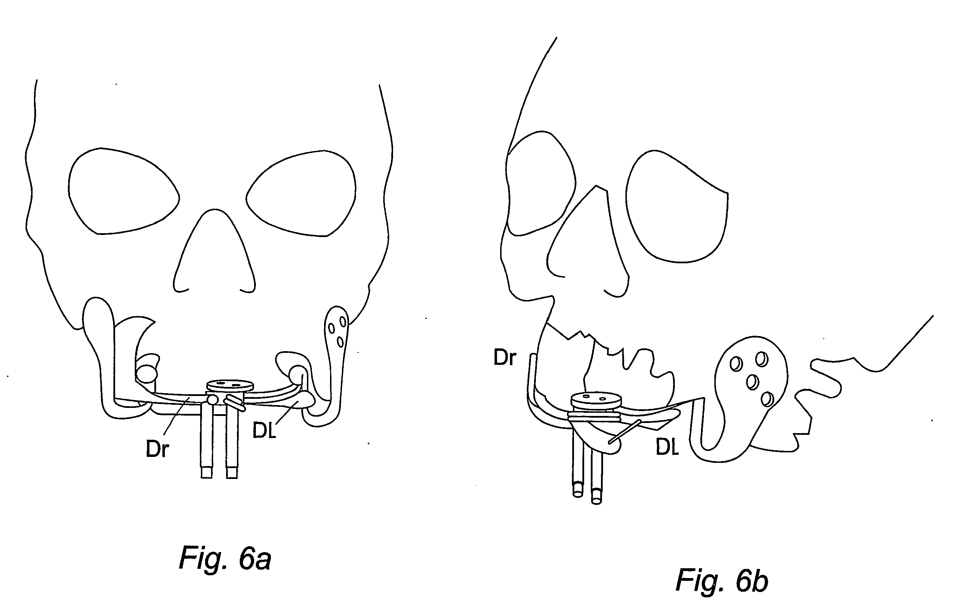

Figures 6a and 6b show front and side views of Figure 5;

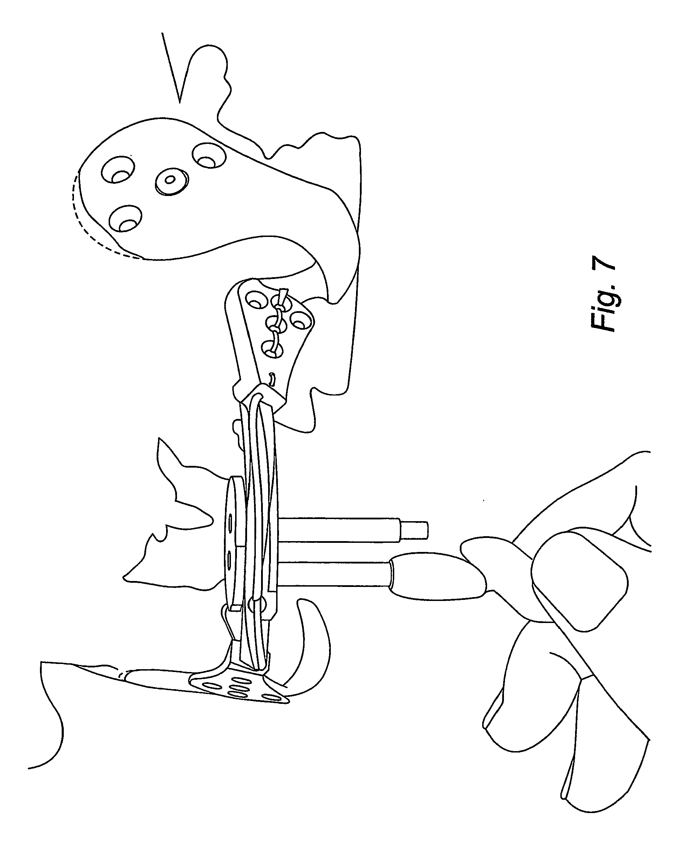

Figure 7 shows activation of the device of previous Figures; and

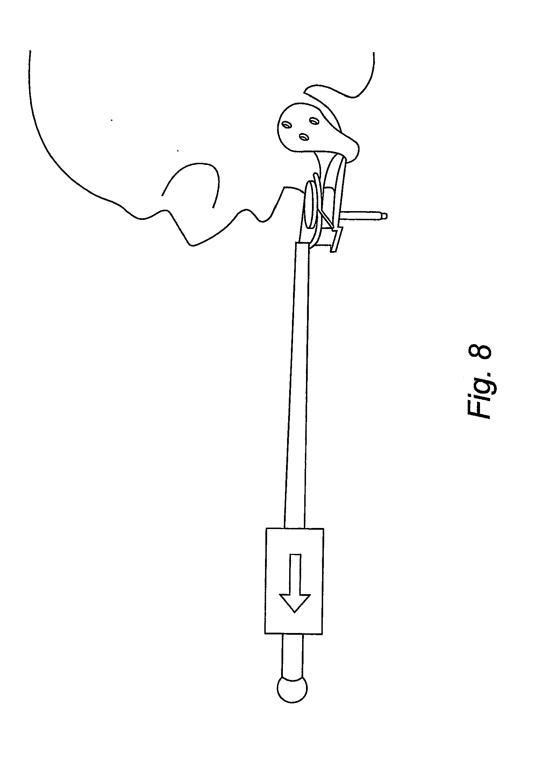

Figure 8 shows extraction of the device after use.

Referring to the drawings:

### Figure 1:

### Disassembled System

### Anchorage to mandibular ramus (A)

Two custom made devices are manufactured using rapid prototyping and reverse engineering techniques. These devices are screwed to the outside of the mandibular ramus with 2.7 mm screws or another appropriate screw system. The design may be planned on a stereoscopic lithographic model (or other engineered model from biological scan data) and loop under the lower border of the mandible to the lingual side of the mandible. Located on the lingual side of the anchorage device is a female part of an attachment device (B) which fits into the male attachment part of the spiral vector bar. This device provides skeletal anchorage for the distraction mechanism. It is possible that this anchorage device could be mass produced.

### Figure 2:

### The Spiral Vector Bar (c)

The spiral vector bar is custom made (but could be mass produced) and consists of an implantable metal bar which slots into the anchorage; as shown later. The principle of this device is that it produces a curved shape that is larger than the patient's anatomical mandible contour, so that when the device is removed the right and left sides of the regenerated bone, the bone can be bent backwards to produce an overlap of bone which may be lag screwed or otherwise fixed to achieve bony union. The lag screw mechanism could incorporate an overdenture abutment attachment which penetrates the oral mucosa for attachment of an overdenture.

The spiral vector bar (C) receives runner bars from one or more mobile shuttles (D), into one or more spiral channels which will be attached to mobilised osteotomised fragments of the posterior mandible. This permits smooth passage of the shuttle around a predetermined vector. A modification of attachment of the shuttle involves the incorporation of a ring which surrounds the spiral vector bar guiding the shuttle on a spiral thread.

The shuttles are sharp to provide a cutting mechanism to facilitate the transport the bone pieces of the shuttle through the body tissues.

A capstan device is located anteriorly (E) with two optional removable key attachments. These are inserted into the capstan using the key (G) and an angled screwdriver (H). The key attachments penetrate through the skin below for daily cranking with a predetermined traction distance. The cranking rod is designed to pull the contralateral shuttle round the spiral to the end point using a traction cord of dacron or other suitable implantable material wire or chain.

A variation of the capstan device includes one or more intra oral key attachments attached directly into the capstan in cases with adequate access and good mouth opening.

An elastic band (I) is shown connecting the shuttles. This is only to stabilise movement of the device in the photographic demonstration of vector dynamics. The elastic band is not included in the device for implantation.

### Figures 3 and 4:

### Key Attachments

The removable key attachments are fixed to the device as illustrated. In this case the capstan is exposed and located in the floor of the mouth.

The key attachments penetrate the skin below the chin.
(I) - indicates the elastic band, (used for photographic illustration of vector dynamics only).

One or more,(in the case illustrated two) traction cords (J) run in a groove within the spiral vector bar and then pass backwards through a hole.

### Figure 5:

### The Assembled System

The device is designed to grow the mandible with a greater anterior projection so that on removal of the device the jaw can be bent backwards to achieve bony contact between the two distracted segments so that the end point is the correct contour of the chin and lower jaw.
A - Skeletal Anchorage Device to mandibular ramus
B - Female attachment part for spiral vector bar
C- Spiral vector bar for choice of selective vector
D - Shuttle
E- Capstan
F - Removable Key attachments

### Figures 6a and 6b:

### End point - Spiral Distraction

The shuttles have been advanced to their endpoint with the creation of a neo-mandible. For illustration the shuttle related to the patient's right side of mandible (Dr) has moved superiorly and its lateral surface is now facing upwards. The Shuttle related to the patient's left side of mandible (Dl) has spiralled inferiorly with its lateral surface now facing inferiorly. This facilitates the removal of the screws into the adjacent bone and permits simple removal of the spiral vector bar with an anterior path of removal between the regenerated pieces of new bone using a ring hammer.

### Figure 7:

### Spiral Vector Distraction

Movement of the key provides the spiral path of distraction on the left side.

The shuttle has razor sharp edges to cleave through the tissues.

### Figure 8:

### Removal of Device

At the end point of spiral distraction, the key attachments are removed.

An appropriate incision is made to expose the anterior aspect of the spiral distractor.

The shuttle screws are removed from the adjacent bone in the leading edge of the neo-mandible.

A specially designed ring hammer is hooked around the back of the capstan and with a sharp tap the spiral vector bar is removed with an anterior path of withdrawal.

The skeletal anchorage devices to the mandibular ramus are then removed through appropriate incisions in the neck.

The mandible at this stage is oversized, and the anterior free bone fragments are bent towards the midline until they overlap, at which point they are screwed together with a position screw or lag screw or screws. This fixation screw device could incorporate an overdenture attachment system which perforates the oral mucosa for denture attachment.

### Stabilisation

Following removal of the spiral vector bar, the new chin position is stabilised using two additional dental implant precision attachment devices /overdenture attachments in the proximal mandible posterior to the osteotomy. Acting with the midline overdenture attachment this gives three point fixation until the malleable callous matures into stable bone over an estimated three month period.

## Claims

1. A spiral distraction device comprising anchoring means (A) for attachment of the device to each side of a patient's existing mandible, bar means (C) conjoined to and intermediate between each side of the patients' mandible and adapted to attach to and receive runner bars from one or more mobile shuttles (D) into one or more spiral channels which will be attached to mobilised osteomised fragments of the posterior mandible such that the mandible contour produced is larger than the patient's anatomical mandible contour, and means to action the intermediate bar means (C) to effect said transport.

2. A device as claimed in Claim 1 wherein the intermediate bar means (C) includes a capstan means (E) through or around which the relevant part of the bar means (C) and/or means for attachment is wound or otherwise rotated to effect the transportation action.

3. A device as claimed in Claim 2 wherein the capstan (E) includes one or more key attachment means (G).

4. A device as claimed in Claim 3 wherein the key attachment means (G) has an aperture in which a key can be inserted.

5. A device as claimed in any one of the preceding Claims wherein the intermediate bar means (C) includes one or more bars comprising the one or more spiral channels wherein the distal ends of the bars are attachable to the mandible anchoring means (A).

6. A device as claimed in any one of the preceding Claims wherein the one or more shuttles (D) are adapted to wholly or substantially cut or cleave through patient's tissue to assist in transportation of the moving bone pieces therethrough.

7. A device as claimed in any one of the preceding Claims wherein the one or more shuttle (D) involves the incorporation of a ring which surrounds the bar comprising one or more spiral channels guiding the shuttle (D) on a spiral thread.

8. A device as claimed in any one of the preceding Claims wherein the anchoring means (A), intermediate bar means (c) and means to action the intermediate bar are separable.

9. A device as claimed in any one of the preceding Claims wherein the bar comprising one or more spiral channels is spiral curved which produces a class 111 or mandible prognathism contour.

10. A device as claimed in any one of the preceding Claims wherein the device also acts as an overdenture attachment system.

## Patentansprüche

1. Spiralförmige Distraktionsvorrichtung, umfassend Verankerungsmittel (A) für die Befestigung der Vorrichtung an jeder Seite eines Kiefers bei einem Patienten, Bogenmittel (C), die mit jeder Seite und zwischen den Seiten des Kiefers des Patienten liegend verbunden sind und die geeignet sind, an Gleitgestängen einer oder mehrerer beweglicher Pendelvorrichtungen (D) befestigt zu werden und diese in einer oder mehreren spiralförmigen Führungen aufzunehmen, die an mobilisierten osteotomierten Fragmenten des hinteren Kiefers befestigt werden, so dass die geschaffene Kieferkontur größer als die anatomische Kieferkontur des Patienten ist, und Mittel, um die dazwischen liegenden Bogenmittel (C) zu betätigen, um diesen Transport zu bewirken.

2. Vorrichtung nach Anspruch 1, wobei das dazwischen liegende Bogenmittel (C) ein Spillmittel (E) umfasst, durch oder um welches der relevante Abschnitt der Bogcnmittel (C) und/oder der Mittel zur Befestigung gewunden wird oder auf andere Weise aufgedreht wird, um den Transportvorgang zu bewirken.

3. Vorrichtung nach Anspruch 2, wobei das Spillmittel (E) ein oder mebrere Schlüsselbefestigungsmittel (G) umfasst.

4. Vorrichtung nach Anspruch 3, wobei das Schlüsselbefestigungsmittel (G) eine Öffnung hat, in die ein Schlüssel eingeführt werden kann.

5. Vorrichtung nach irgendeinem der vorstehenden Ansprüche, wobei das dazwischen liegende Bogenmittel (C) einen oder mehrere Bögen einschließt, die die eine oder mehreren gewundenen Führungen umfassen, wobei die distalen Enden der Bögen an den Kieferverankerungsmitteln (A) befestigbar sind.

6. Vorrichtung nach irgendeinem der vorstehenden Ansprüche, wobei eine oder mehrere Pendelvorrichtungen (D) geeignet ist/sind, das Gewebe des Patienten ganz oder teilweise zu durchschneiden oder zu zerteilen, um den Transport der beweglichen Knochenteile hierdurch zu unterstützen.

7. Vorrichtung nach irgendeinem der vorstehenden Ansprüche, wobei die eine oder mehreren Pendelvorrichnmgen (D) das Aufnehmen eines Rings einschließt, der den Bogen umgibt, der eine oder mehrere spiralförmige Führungen umfasst, die die Pendelvorrichtung (D) entlang eines Spiralgewinde führen.

8. Vorrichtung nach irgendeinem der vorstehenden Ansprüche, wobei die Verankerungsmittel (A), die dazwischen liegenden Bogenmittel (C) und Mittel zur Betätigung des dazwischen liegenden Bogens, trennbar sind.

9. Vorrichtung nach irgendeinem der vorstehenden Ansprüche, wobei der Bogen, der eine oder mehrere spiralförmige Führungen umfasst, spiralförmig gekrümmt ist, was eine Kontur nach einer Klasse 111 oder einer Kieferprognathie schafft.

10. Vorrichtung nach irgendeinem der vorstehenden Ansprüche, wobei die Vorrichtung auch als ein Befestigungssystem für eine Overlay-Prothese dient.

## Revendications

1. Dispositif d'exognathie à spirale comprenant des moyens d'ancrage (A) pour attacher le dispositif de chaque côté d'un maxillaire existant d'un patient, des moyens en forme de barre (C) réunis de manière intermédiaire entre chaque côté du maxillaire du patient et adaptés à être attachés et recevoir des barres déplaçables depuis une ou plusieurs navettes mobiles (D) vers un ou plusieurs canaux en spirale qui seront attachées à des fragments mobilisés d'ostéotomie du maxillaire postérieur, de sorte que le contour de maxillaire produit est plus grand que le contour anatomique du maxillaire du patient, et des moyens pour agir sur les moyens en forme de barre intermédiaire (C) pour effectuer ledit transport.

2. Dispositif selon la revendication 1, dans lequel les moyens en forme de barre intermédiaire (C) incluent des moyens à cabestan (E) à travers lesquels ou autour desquels la partie concernée des moyens en forme de barre (C) et/ou des moyens d'attache, est enroulée ou mise en rotation d'une autre manière pour effectuer l'action de transport.

3. Dispositif selon la revendication 2, dans lequel le cabestan (E) inclut un ou plusieurs moyens d'attache à clé (G).

4. Dispositif selon la revendication 3, dans lequel les moyens d'attache à clé (G) présentent une ouverture dans laquelle une clé peut être introduite.

5. Dispositif selon l'une quelconque des revendications précédentes, dans lequel les moyens en forme de barre intermédiaire (C) incluent une ou plusieurs barres comprenant lesdits un ou plusieurs canaux en spirale, et dans lequel les extrémités distales des barres peuvent être attachées aux moyens d'ancrage de maxillaire (A).

6. Dispositif selon l'une quelconque des revendications précédentes, dans lequel lesdites une ou plusieurs navettes (D) sont adaptées pour couper totalement ou sensiblement à travers les tissus du patient pour aider au transport des pièces osseuses en déplacement à travers lesdits tissus.

7. Dispositif selon l'une quelconque des revendications précédentes, dans lequel lesdites une ou plusieurs navettes (D) incorporent une bague qui entoure la barre et comprenant un ou plusieurs canaux en spirale qui guident la navette (D) sur un trajet en spirale.

8. Dispositif selon l'une quelconque des revendications précédentes, dans lequel les moyens d'ancrage (A), les moyens en forme de barre intermédiaire (C), et les moyens pour actionner la barre intermédiaire, sont séparables.

9. Dispositif selon l'une quelconque des revendications précédentes, dans lequel la barre comprenant un ou plusieurs canaux en spirale est incurvée en forme de spirale, ce qui produit un contour de classe 111 ou contour de maxillaire prognathe.

10. Dispositif selon l'une quelconque des revendications précédentes, dans lequel le dispositif fait aussi office de système d'attache superposé à la denture.
